# FASCICULE DE BREVET EUROPEEN

(11) **EP 3 717 658 B1**
(45) Date de publication et mention de la délivrance du brevet: **21.06.2023**
(21) Numéro de dépôt: 18827199.3
(22) Date de dépôt: 29.11.2018
(51) Int. Cl.: C12Q 1/14, C12M 1/12, C12Q 1/34, A61B 5/145, A61B 5/1486, C12Q 1/04, C12Q 1/18, A61B 5/00

(54) **APPAREILS DE DÉTECTION ET/OU D'IDENTIFICATION DES INFECTIONS MICROBIOLOGIQUES POUR DISPOSITIFS MÉDICAUX NON IMPLANTABLES ET COMPOSITION DE POUDRES**
GERÄTE ZUR ERFASSUNG UND / ODER IDENTIFIZIERUNG VON MIKROBIOLOGISCHEN INFEKTIONEN FÜR NICHT IMPLANTIERBARE MEDIZINPRODUKTE UND PULVERZUSAMMENSETZUNG
DEVICES FOR THE DETECTION AND/OR IDENTIFICATION OF MICROBIOLOGICAL INFECTIONS FOR NON-IMPLANTABLE MEDICAL DEVICES AND POWDER COMPOSITION

(30) Priorité: 30.11.2017 FR 1761403
(43) Date de publication de la demande: 07.10.2020
(73) Titulaire: bioMérieux, 69280 Marcy-l'Étoile (FR)
(72) Inventeur: BERRAT, Fanny, 69160 Tassin La Demi Lune (FR); DARDY, Aline, 69009 Lyon (FR); ROZAND, Christine, 69290 Saint Genis Les Olliers (FR)
(74) Mandataire: bioMérieux PI Groupement mandataires
(86) Numéro de dépôt international: PCT/FR2018/053034
(87) Numéro de publication internationale: WO 2019/106297

(56) Documents cités:
- WO-A1-2010/022111
- WO-A2-2012/166848
- US-A- 5 635 367
- US-A- 6 090 541

## Description

La présente invention appartient au domaine du diagnostic microbiologique et trouve une application dans celui des dispositifs médicaux non-implantables utilisés pour recouvrir les plaies et les lésions cutanées en vue de les protéger des souillures et des infections extérieures, tels que pansements, compresses et autres articles absorbants analogues. La présente invention a plus précisément trait à des moyens de diagnostic d'infections microbiologiques, destinés à être embarqués dans de tels dispositifs médicaux non-implantables, en vue de détecter la survenue d'une éventuelle infection.

Les plaies, quelle que soit leur gravité, sont soumises à des risques d'infection par des microorganismes pathogènes tels que bactéries, virus, champignons, protozoaires. Outre le fait d'interrompre le processus physiologique de cicatrisation, ces infections peuvent entrainer des complications sérieuses, telles que gangrène gazeuse, tétanos, et donner lieu à des incapacités à long terme (infection chronique de la plaie ou de l'os. ..), voire à un décès consécutif à un sepsis.

Dans le cas des hospitalisations, plus de 90 % des infections postopératoires constatées surviennent dans les 30 jours qui suivent un acte chirurgical. Les patients doivent ainsi être suivis au moins durant les 30 jours après avoir été opérés. Cliniquement, ce suivi consiste généralement en un examen visuel de la cicatrisation des plaies et en la constatation d'un éventuel écoulement purulent qui -selon la couleur, la consistance et l'odeur- peut être le signe d'une infection. Lorsqu'une infection est suspectée, un prélèvement est réalisé puis expédié au laboratoire d'analyse pour des investigations complémentaires : analyses microscopiques, cultures microbiologiques et/ou antibiogrammes... En pratique, le praticien ne disposera des résultats d'analyses que le lendemain, au plus tôt.

Pour un meilleur suivi des patients, les professionnels de la santé (médecins, praticiens, infirmières...) ont émis le souhait de pouvoir disposer de tests et d'outils de diagnostic leur permettant de constater par eux-mêmes l'existence d'une infection et, éventuellement envisager le traitement à prescrire lors de la consultation même.

Dans ce contexte, il a été envisagé d'intégrer des moyens de détection et/ou de diagnostic d'infections microbiologiques dans la structure des compresses, pansements et autres dispositifs médicaux non-implantables utilisés pour recouvrir et protéger les plaies.

Dans une première approche, sur la base des récentes avancées technologiques dans le domaine des microélectrodes, des capteurs électrochimiques ont été développés pour réaliser des analyses électrochimiques *in situ* de la composition chimique des exsudats des plaies et ce, en vue de détecter la présence de biomarqueurs associés à des infections microbiologiques.

Dans ce contexte, on peut notamment citer les travaux de Hajnsek et al., 2015 (SENSOR AND ACTUATORS B: CHEMICAL ; 265-274 : « An electrochemical sensor for fast détection of wound infection based on myeloperoxidase activity ») et Sismaet et al., 2016 (WOUND REPAIR AND REGENERATION; (24)366-372 : «Electrochemical détection of Pseudomonas in wound exudate samples from patients with chronic wounds »). Dans le premier exemple, le capteur permet la détection électrochimique d'une activité myéloperoxidase, qui est exprimée par de nombreuses bactéries pathogènes. Dans le deuxième exemple, le capteur permet une détection électrochimique de la pyocyanine, une molécule produite par *Pseudomonas aeruginosa.*

Ces capteurs électrochimiques sont aujourd'hui à un stade expérimental précoce de leur développement. Ils embarquent une technologie d'électrodes miniaturisées dont le coût actuel est incompatible avec le marché des pansements et des compresses. Par ailleurs, de tels capteurs électrochimiques ne sont pas autonomes. Pour fonctionner, ils doivent être connectés à tout un équipement externe apte à les alimenter en électricité, à recueillir les données transmises par les capteurs, et à les traiter avant de pouvoir rendre un résultat d'analyse au praticien ; autant d'équipements que ce dernier devra avoir à sa disposition.

Dans une deuxième approche, davantage en accord avec les contraintes technologiques et économiques du marché des pansements et des compresses, le concept d'un pansement intelligent (ou « *smart bandage* » en anglo-saxon) a été imaginé. Posé sur la plaie, celui-ci génèrerait un signal visible (apparition d'une couleur et/ou d'une fluorescence) en cas de survenue d'une infection microbiologique.

Dans ce contexte, Brocklesby et al., 2013 (Medical hypothèses ; (80)237-240 : « Smart bandages - A colourful approach to early stage infection détection & control the wound care ») envisage la réalisation d'un pansement permettant la détection des bactéries productrices de *β-*lactamases. Pour ce faire, il est suggéré d'utiliser une céphalosporine « chimérique » chromogène, assemblée à partir d'un résidu de céphalosporine et d'un chromophore. Cette céphalosporine « chimérique » chromogène serait fixée grâce à un couplage chimique aux fibres du textile absorbant constitutif du pansement. Son hydrolyse par des céphalosporinases provoquerait la coloration du pansement. A ce jour, aucun dispositif ou pansement fonctionnant sur ce principe de détection n'a vu le jour.

Plus récemment, les chercheurs de l'Université de Bath au Royaume-Uni ont annoncé la mise au point d'un prototype de pansement intelligent émettant une fluorescence en réponse à une infection bactérienne (Thet et al., 2016 - ACS Applied Materials & Interfaces ; 8(24): 14909-19 : «Prototype development of the intelligent hydrogel wound dressing and its efficacy in the détection of model pathogenic wound biofilm »).

Ce pansement est formé d'une pièce de tissu absorbant, dont la face destinée à être appliquée au contact-même de la plaie du patient, est recouverte d'un hydrogel d'agarose 2 %. Dans la masse de cet hydrogel, se trouvent réparties des vésicules chargées de 5,6-carboflurescéine. La paroi de ces vésicules est constituée d'un assemblage de phospholipides, de cholestérol et de polymères de polyacétylène. Par sa composition, elle mime la membrane phospholipidique des cellules eucaryotes et est perforée par les cytotoxines que les bactéries pathogènes relarguent au cours d'une infection. La 5,6-carboflurescéine, quant à elle, est un composé qui présente deux particularités intéressantes, ingénieusement exploitées dans ce dispositif. La première est qu'elle s'autoassemble en dimères, lorsque sa concentration est suffisamment élevée, puis se dissocie au fur et à mesure que sa concentration diminue. La deuxième particularité est qu'elle émet une fluorescence lorsqu'elle est à l'état monomérique ; à l'état de dimère, sa fluorescence est par contre bloquée.

En cas d'infection bactérienne, la paroi des vésicules est rompue par les cytotoxines relarguées, et la 5,6-carboflurescéine est libérée des vésicules et se dilue progressivement dans l'hydrogel. Exposé à une lumière UV, le pansement émet alors une fluorescence.

Actuellement, ce dispositif est au stade de prototype et de nombreux tests sont prévus pour s'assurer de l'innocuité de l'hydrogel d'agarose, de celle des vésicules et de la 5,6-carboflurescéine, qui se retrouveront directement au contact de la plaie du patient.

Un tel dispositif connait quelques inconvénients majeurs. Un premier inconvénient est lié à la non-spécificité de la détection. Un tel dispositif ne pourra qu'alerter le praticien sur la survenue d'une infection. Des tests complémentaires devront être réalisés, s'il souhaite connaître la nature du(es) pathogène(s) incriminé(s) et envisager la prescription d'un traitement spécifique. Un deuxième inconvénient vient de la stabilité dans le temps de l'hydrogel d'agarose et relève davantage des considérations propres à l'exploitation industrielle et commerciale d'un tel dispositif. Du fait de la présence de cet hydrogel, le succès commercial d'un tel dispositif pâtira d'une durée de péremption courte, d'importantes contraintes de conditionnement et de conservation.

La présente invention vise à résoudre les inconvénients précédemment énoncés. Elle a ainsi pour but général de proposer une solution technique à la réalisation de pansements dits intelligents, notamment en termes de simplicité de mise en oeuvre et de spécificité de la détection.

Un objectif de la présente invention est de proposer des moyens de diagnostic d'infections microbiologiques pouvant être intégrés principalement à des structures de type pansement, compresse et autres articles absorbants destinés à recouvrir les plaies et les lésions cutanées, et pourraient signaler un état d'infection bactérienne par un changement de couleur et/ou par l'émission d'une fluorescence. Un autre objectif de la présente invention est de permettre une détection et une identification spécifique du(des) pathogène(s) en cause, grâce à l'apparition d'un profil de coloration et/ou d'émission de fluorescence, particuliers et caractérisables.

La présente invention a pour ambition de proposer des moyens de diagnostic *in situ* d'infections microbiologiques, pouvant être transposables/applicables à l'industrie des pansements, compresses... mais également à des domaines techniques voisins (en particulier, ceux des articles absorbants du type couche-culotte et serviette hygiénique, utilisés à des fins médicales) et ce, de façon compatible avec les contraintes d'une exploitation industrielle et commerciale propres à ce secteur.

Enfin, la présente invention a pour vocation de proposer une technologie qui puisse être exploitée aussi bien pour la médecine humaine que pour la médecine vétérinaire.

Dans ce contexte, la présente invention propose des moyens de diagnostic *in situ* d'infections microbiologiques se présentant sous la forme d'un biocapteur comprenant une pièce en matériau absorbant et hydrophile fixant, en surface et/ou dans son épaisseur, une composition de poudres agglomérées comprenant des particules d'éthylène-acétate de vinyle (ou EVA, pour « *ethylene-vinyl acetate* » en anglo-saxon) ayant une surface en partie recouverte au moins d'un sel d'acide orthophosphorique et d'un indicateur visuel de croissance microbiologique. En présence de microorganismes, en particulier de bactéries et de levures, ledit indicateur visuel de croissance microbiologique est apte à produire un signal visible à l'oeil lorsque le biocapteur est exposé à la lumière blanche et/ou à une radiation UV.

Un biocapteur selon l'invention est destiné à être intégré dans la structuremême d'un pansement, d'une compresse ou de tout autre article absorbant analogue permettant de recouvrir les plaies et les lésions cutanées. Positionné à proximité d'une plaie ou d'une lésion, le biocapteur est conçu pour pouvoir capter et recueillir l'exsudat des plaies, et former un support propice à la croissance et au développement de microorganismes. La détection et/ou l'identification des microorganismes éventuellement présents s'effectuent grâce aux indicateurs visuels (chromogènes et/ou fluorogènes) de croissance microbiologique, sensibles à l'activité métabolique des microorganismes recherchés. Le biocapteur de la présente invention est défini dans la revendication 1.

Pour concevoir un tel biocapteur, les inventeurs ont mis à profit les techniques de microbiologie *in vitro* visant la détection et l'identification de microorganismes tels que des bactéries, levures, moisissures et amibes, et se sont plus particulièrement inspirés des travaux menés depuis les années 90 sur les milieux de culture chromogéniques et fluorogéniques (Perry et al., 2007 - Journal of Applied Microbiology ; 103:2046-55 : « The application of chromogenic media in clinical microbiology »).

De la composition de ces milieux de culture, les inventeurs ont essentiellement repris et appliqué les enseignements dispensés sur les indicateurs visuels de croissance microbiologique, dont l'utilisation permet une mise en évidence visuelle des microorganismes via leur activité métabolique. On trouve dans la littérature et dans le commerce, de nombreux indicateurs visuels de croissance microbiologique, susceptibles de pouvoir être utilisés pour confectionner un biocapteur selon l'invention. Il peut s'agir, par exemple, d'indicateurs colorés de pH, sensibles à la variation de pH induite par une hydrolyse de sucres ou d'autres substrats métabolisables par les microorganismes ciblés. Plus préférentiellement, il s'agit de substrats chromogènes et/ou fluorogènes aptes à libérer un composé chromophore et/ou fluorophore sous l'action d'une activité enzymatique particulière (telle que par exemple, les activités β-glucuronidase, β-galactosidase, β-glucosidase, α-glucuronidase, α-galactosidase, α-glucosidase, estérase, nitroréductase, phosphatases, aminopeptidase...).

Pour éliminer tout risque de surinfection de la plaie qui pourrait être imputable au biocapteur, les inventeurs ont pris soin, dans la conception de ce dernier, de bannir tout ingrédient ayant un potentiel nutritif à l'égard des microorganismes. Avantageusement, la composition de poudres agglomérées fixée dans le biocapteur selon l'invention ne présente aucune qualité nutritive notable/mesurable pour la croissance et le développement des microorganismes. En particulier, cette composition de poudres agglomérées est exempte de toute quantité physiologiquement effective de glucides, de lipides, d'acides aminés et de facteurs de croissance, apportés sous forme de compositions chimiquement bien définies ou sous la forme de compositions complexes telles que des mélanges de peptones, des extraits de levures, des sérums...

Ainsi, avec un biocapteur selon l'invention, en cas d'infection microbiologique des plaies, les microorganismes ne peuvent puiser les nutriments nécessaires à leur développement que dans l'exsudat de la plaie, seule source nutritive disponible dans cet environnement particulier.

Pour embarquer et immobiliser dans le matériau absorbant une quantité d'indicateur visuel de croissance microbiologique qui soit opérante pour une détection et/ou une identification des microorganismes cibles, il a fallu chercher un adhésif pouvant répondre aux trois conditions majeures suivantes :
- être capable de fixer efficacement les indicateurs visuels de croissance microbiologique au matériau absorbant et hydrophile, sans nuire à leur fonctionnement ni gêner leur hydrolyse en cas d'infection par des microorganismes ciblés,
- être d'une efficacité suffisante pour que la quantité à utiliser ne soit pas dommageable pour les capacités d'absorption du matériau absorbant et hydrophile,
- être physiologiquement neutre à l'égard des microorganismes, c'est-à-dire ne présenter ni d'effets activateurs ou inhibiteurs notables sur la croissance et le développement des microorganismes.

Aucun des matériaux adhésifs testés par les inventeurs n'a pu satisfaire pleinement à ces trois conditions. Néanmoins, parmi les nombreux matériaux testés, l'EVA a suscité un grand intérêt auprès des inventeurs.

Résine synthétique bien connue depuis les années 50 pour ses propriétés thermoplastiques et thermodurcissables, l'EVA est préparé par copolymérisation de l'éthylène avec l'acétate de vinyle (la teneur pondérale d'acétate de vinyle varie généralement entre 10 et 40 %). On le retrouve sous des formes très diverses dans de nombreux secteurs de l'industrie (par exemple, un film plastique, un adhésif pour le thermoscellage d'emballages, une colle dans la reliure des livres, une couche superficielle du papier glacé des magazines, un plastifiant dans les compositions de vernis et de peinture, un composant d'un matériau plastique thermo-moulé, un composant d'une composition d'encapsulation de principes actifs...). Dans le cadre de la mise au point des biocapteurs selon l'invention, les inventeurs ont pu constater une manipulation et une utilisation de l'EVA d'une grande simplicité. Un chauffage à des températures de l'ordre de 50-60°C permet de ramollir ces particules et rendre leur surface collante. Portées à de telles températures, les particules d'EVA présentent une bonne adhérence de surface pour un grand nombre de composés, en particulier pour les particules d'indicateurs visuels de croissance microbiologique qu'elles peuvent fixer et exposer à leur surface. Malheureusement, les inventeurs ont aussi constaté une activité bactériostatique de l'EVA, le rendant rédhibitoire pour une application dans le domaine de la microbiologie et d'autant plus, lorsque la finalité est une culture cellulaire à des fins de détection et/ou d'identification. Cependant, à force de persistance, les inventeurs sont parvenus à surmonter cet obstacle et ont démontré que l'adjonction d'un sel d'acide orthophosphorique permet d'inhiber cet effet bactériostatique indésirable.

Selon l'invention, le(les) sel(s) d'acide orthophosphorique utilisé(s) pour inhiber l'effet bactériostatique de l'EVA est(sont) choisi(s) parmi le dihydrogénophosphate de potassium (KH₂PO₄) et le dihydrogénophosphate de sodium (NaH₂PO₄).

Selon l'invention le dihydrogénophosphate de potassium est utilisé pour inhiber l'effet bactériostatique de l'EVA. Le rapport pondéral KH₂PO₄/EVA est compris entre 1:25 et 1:8, préférentiellement compris entre 1:16 et 1:12.

Selon l'invention le dihydrogénophosphate de sodium est utilisé pour inhiber l'effet bactériostatique de l'EVA. Le rapport pondéral NaH₂PO₄/EVA est compris entre 1:15 et 1:3, préférentiellement compris entre 1:10 et 1:5.

Selon un troisième mode de réalisation, pour inhiber l'effet bactériostatique de l'EVA, KH₂PO₄ et NH₂PO₄ sont utilisés concomitamment.

Avantageusement et selon l'invention, l'EVA présente une teneur pondérale en acétate de vinyle de 10 à 40 %, préférentiellement de l'ordre de 20 à 35%.

Avantageusement et selon l'invention, la surface des particules d'EVA est également recouverte en partie d'un agent gélifiant qui, après absorption de l'exsudat, donne un gel. Utiliser un agent gélifiant dans ce contexte particulier présente plusieurs intérêts. En premier lieu, il améliore les performances d'absorption de la pièce en matériau absorbant et hydrophile, qui entre dans la constitution du biocapteur. Egalement, il permet de retenir et de bloquer en position les microorganismes captés.

Nombre d'agents gélifiants peuvent être utilisés à cette fin, notamment l'agar, l'agarose, la gomme de guar et la gomme de xanthane. La gomme de xanthane est préférée.

Selon un mode de réalisation particulier d'un biocapteur selon l'invention, la surface des particules d'EVA peut aussi être en partie recouverte d'un agent sélectif. Cet agent sélectif est choisi pour bloquer ou ralentir le développement de la flore annexe, plutôt que celle d'un microorganisme-cible ou d'un groupe-cible de microorganismes. Il s'agit essentiellement de composés aux effets antibiotiques et/ou antifongiques, ayant une certaine spécificité de toxicité (toxicité plus faible pour les microorganismes-cibles que pour la flore annexe).

S'agissant de la pièce en matériau absorbant et hydrophile constitutive d'un biocapteur objet de la présente invention, de nombreux matériaux peuvent être utilisés. On utilise avantageusement un matériau fibreux, composé de fibres non-tissées, formant un ensemble ayant une intégrité et une cohérence mécanique. Lesdites fibres peuvent être des fibres cellulosiques naturelles (comme le coton) ou synthétiques (comme la rayonne), des fibres de cellulose modifiée (par exemple, la carboxyméthylcellulose, la nitrocellulose), des fibres de polymères chimiques (tels que les sels de polyacrylate, les copolymères acrylate/acrylamide).

Avantageusement, ladite pièce en matériau absorbant présente une masse surfacique comprise entre 50 g.m⁻² et 200 g.m⁻², pour une épaisseur avantageusement comprise entre 0,5 mm et 4 mm.

Selon un mode de réalisation préféré, cette pièce en matériau absorbant et hydrophile est réalisée à partir d'un textile non-tissé, par exemple à base de fibres de cellulose. Selon ce mode préféré, la composition de poudres agglomérées est avantageusement fixée aux fibres du matériau, essentiellement dans son épaisseur.

L'incorporation d'une composition sèche dans l'épaisseur d'un substrat fibreux peut être réalisée de diverses façons, notamment par des techniques dites d'imprégnation à sec. A cet effet, une composition sèche et pulvérulente est saupoudrée sur la surface d'un substrat poreux, puis les particules de ladite composition sont mises en vibration, sous l'action d'ondes ultrasonores (cf. par exemple, FR 2 866 578) ou bien sous l'action d'un champ électrique alternatif (cf. par exemple, WO 2015/044605, WO 2010/001043 ou WO 99/22920). Ces particules pénètrent et s'enfoncent alors progressivement dans les cavités du corps poreux.

Pour la fabrication d'un dispositif de culture microbiologique selon l'invention, les techniques d'imprégnation à sec mettant en oeuvre un champ électrique alternatif se sont révélées particulièrement adaptées pour permettre l'incorporation d'une poudre dans l'épaisseur d'un matériau absorbant hydrophile, ayant une structure fibreuse. De ce fait, les enseignements techniques dispensés par WO 2015/044605, WO 2010/001043 et WO 99/22920 font partie intégrante de la présente description.

Avantageusement, la quantité de composition de poudres agglomérées incorporée au bioréacteur selon l'invention équivaut à une concentration comprise entre 10 mg.cm⁻³ et 100 mg.cm⁻³, préférentiellement entre 30 mg.cm⁻³ et 50 g.cm⁻³.

Une fois l'imprégnation à sec réalisée, un traitement thermique permet de rendre collant l'EVA et de fixer les particules de poudres agglomérées aux fibres du substrat absorbant. Ce traitement thermique consiste avantageusement en une opération de calandrage. Le calandrage, par la pression et la température de chauffage appliquées, permet de fixer et de retenir durablement, dans l'épaisseur de la pièce en matériau absorbant et hydrophile, les particules d'EVA avec l'(es) indicateur(s) visuel(s) de croissance microbiologique exposés à leur surface. Le calandrage améliore également la planéité de la surface de la pièce en matériau absorbant et hydrophile et accroit ses capacités de capillarité et d'absorption.

Le calandrage se fait avantageusement à une température comprise entre 50°C et 80°C.

La présente invention s'étend également à une composition de poudres agglomérées. Ladite composition de poudres agglomérées comprend des particules d'EVA ayant une surface en partie recouverte au moins d'un sel d'acide orthophosphorique et d'un indicateur visuel de croissance microbiologique.

Selon l'invention, le(les) sel(s) d'acide orthophosphorique est(sont) choisi(s) parmi le dihydrogénophosphate de potassium (KH₂PO₄) et le dihydrogénophosphate de sodium (NaH₂PO₄).

Selon l'invention le dihydrogénophosphate de potassium est utilisé dans rapport pondéral KH₂PO₄/EVA compris entre 1:25 et 1:8, préférentiellement compris entre 1:16 et 1:12.

Selon l'invention le dihydrogénophosphate de sodium est utilisé dans un rapport pondéral NaH₂PO₄/EVA compris entre 1:15 et 1:3, préférentiellement compris entre 1:10 et 1:5.

Les compositions de poudres de la présente invention sont définies dans les revendications 11 et 12.

Selon un troisième mode de réalisation, KH₂PO₄ et NaH₂PO₄ sont concomitamment présents à la surface des particules d'EVA.

Une composition de poudres agglomérées selon l'invention trouve un intérêt tout particulier pour les biocapteurs selon l'invention ; elle en facilite grandement la fabrication.

Avantageusement et selon l'invention, le mélange pulvérulent que forment le(s) sel(s) d'acide orthophosphorique et l'(es) indicateur(s) visuel(s) de croissance microbiologique et dont les particules sont destinées à recouvrir la surface des particules d'EVA, comprend également un agent gélifiant. Ledit agent gélifiant peut être de l'agar, de l'agarose, de la gomme de guar ou de la gomme de xanthane. Selon ce mode de réalisation, la surface des particules d'EVA est également en partie recouverte d'un tel agent gélifiant. La gomme de xanthane est préférée.

Avantageusement et selon l'invention, ce mélange pulvérulent peut aussi comprendre un agent stimulateur du métabolisme des bactéries, par exemple du chlorure de manganèse (MnCl₂), et/ou un agent sélectif aux effets antibiotiques et/ou antifongiques. Selon ce mode de réalisation, la surface des particules d'EVA est également en partie recouverte d'un agent stimulateur du métabolisme des bactéries. MnCl₂ est préféré.

Avantageusement et selon l'invention, les particules d'EVA de la composition de poudres agglomérées selon l'invention présentent une granulométrie comprise entre 40 et 150 µm. Les particules de sel(s) d'acide orthophosphorique, les particules d'indicateur(s) visuel(s) de croissance microbiologique, éventuellement des particules d'agent gélifiant, des particules d'agent stimulateur du métabolisme des bactéries et des particules d'agent sélectif, qui recouvrent la surface de ces particules d'EVA ont une granulométrie avantageusement comprise entre 5 et 50 µm.

L'invention concerne également un biocapteur, une composition de poudres agglomérées ainsi que tout dispositif médical non implantable (tels que pansements, compresses... mais également couches-culottes et serviettes hygiéniques utilisées à des fins de suivi médical humain ou animal) incorporant dans sa structure un tel biocapteur et/ou une telle composition sèche et pulvérulente, caractérisés par tout ou partie des caractéristiques techniques exposées ci-dessus et ci-dessous.

D'autres buts, caractéristiques et avantages de l'invention apparaîtront au vu de la description qui va suivre et des exemples développés ci-après, qui se réfèrent aux figures annexées et dans lesquelles :
- la Figure 1 est une représentation graphique de l'effet bactériostatique de l'EVA sur le développement des bactéries, et de l'inhibition de cet effet bactériostatique par les sels d'acide orthophosphorique ;
- la Figure 2 correspond à deux photographies, prises en microscopie électronique à balayage, montrant une composition pulvérulente de particules dissociées ;
- la Figure 3 correspond à deux photographies, prises en microscopie électronique à balayage, montrant une composition de poudres agglomérées selon l'invention.

### EXEMPLES

### Exemple 1 : Démonstration de l'effet bactériostatique de l'EVA et de l'inhibition de cet effet par addition de sels d'acide orthophosphorique (KH₂PO₄, NaH₂PO₄)

### A. Démonstration réalisée sur des milieux de culture gélosés

Les effets de l'EVA et des sels de l'acide orthophosphorique (plus particulièrement, KH₂PO₄ et/ou NaH₂PO₄) sur la croissance et le développement bactériens ont été évalués, dans un premier temps, sur des milieux de culture gélosés, en l'occurrence :
- une gélose Trypto-caséine soja (TSA), un milieu universel connu comme pouvant convenir à la culture et à l'enrichissement de la plupart des bactéries, qu'elles soient aérobies ou anaérobies ;
- la gélose SAID^{®} (bioMérieux, France), un milieu de culture commercialisé pour la détection spécifique de *S. aureus,*

A cet effet, des souches de *S. aureus* provenant de la collection de la Demanderesse ont été utilisées. Avant de les inoculer aux deux géloses susmentionnées, les bactéries (100 mL d'une suspension de 10³ cfu.mL⁻¹) ont été préalablement resuspendues dans du sérum humain préparé et fourni par l'Etablissement Français du Sang (EFS), complémenté ou non en EVA et/ou en sel d'acide orthophosphorique comme suit :
- s01 : sérum (EFS, référence 4566200 / 4566201)
- s02 : sérum s01 additionné d'EVA (à 53 g.L⁻¹, de concentration finale)
- s03 : sérum s01 additionné d'EVA et de NaH₂PO₄ (respectivement à 53 g.L⁻¹ et 4,5 g.L⁻¹, de concentrations finales)
- s04 : sérum s01 additionné d'EVA et de NaH₂PO₄ (respectivement à 53 g.L⁻¹et 9,0 g.L⁻¹, de concentrations finales)
- s05 : sérum s01 additionné de NaH₂PO₄ (à 4,5 g.L⁻¹, de concentration finale)
- s06 : sérum s01 additionné de NaH₂PO₄ (à 9,0 g.L⁻¹, de concentration finale)
- s07 : sérum s01 additionné d'EVA et de KH₂PO₄ (respectivement à 53 g.L⁻¹ et 10,6 g.L⁻¹, de concentrations finales)
- s08 : sérum s01 additionné d'EVA et de KH₂PO₄ (respectivement à 53 g.L⁻¹ et 1,5 g.L⁻¹, de concentrations finales)
- s09 : sérum s01 additionné d'EVA et de KH₂PO₄ (respectivement à 53 g.L⁻¹et 3,0 g.L⁻¹, de concentrations finales)
- s10 : sérum s01 additionné de KH₂PO₄ (à 1,5 g.L⁻¹, de concentration finale)
- s11 : sérum s01 additionné d'EVA, de NaH₂PO₄ et de KH₂PO₄ (respectivement à 53 g.L⁻¹, 4,5 g.L⁻¹ et 1,5 g.L⁻¹, de concentrations finales)
- s12 : sérum s01 additionné d'EVA, de NaH₂PO₄ et de KH₂PO₄ (respectivement à 53 g.L⁻¹, 9,0 g.L⁻¹ et 3,0 g.L⁻¹, de concentrations finales)
- s13 : sérum s01 additionné de NaH₂PO₄ et de KH₂PO₄ (respectivement à 4,5 g.L⁻¹ et 1,5 g.L⁻¹, de concentrations finales)
- s14 : sérum s01 additionné de NaH₂PO₄ et de KH₂PO₄ (respectivement à 9,0 g.L⁻¹ et 3,0 g.L⁻¹, de concentrations finales).

L'EVA utilisé dans ces essais est commercialisé par la société DAKOTA, Belgique, sous la forme d'une poudre de référence UNEX EVA (EVA T1). Sa teneur pondérale en acétate de vinyle est de 28 %.

Les résultats obtenus sont compilés dans le Tableau 1, ci-après.

**Tableau 1**

| Sérums | TSA | SAID |
|---|---|---|
| s01 | tapis bactérien | tapis bactérien |
| s02 | 1 colonie | Rien |
| s03 | > 300 colonies | > 300 colonies |
| s04 | 300 colonies | 300 colonies |
| s05 | tapis bactérien | tapis bactérien |
| s06 | tapis bactérien | tapis bactérien |
| s07 | rien | Rien |
| s08 | tapis bactérien | tapis bactérien |
| s09 | tapis bactérien | tapis bactérien |
| s10 | tapis bactérien | tapis bactérien |
| s11 | rien | Rien |
| s12 | rien | Rien |
| s13 | tapis bactérien | tapis bactérien |
| s14 | tapis bactérien | tapis bactérien |

### B. Démonstration réalisée sur un support de culture absorbant et hydrophile

L'évaluation des effets de l'EVA et des sels de l'acide orthophosphorique (plus particulièrement, KH₂PO₄) sur la croissance et le développement bactérien a été poursuivie sur un support de culture absorbant et hydrophile, incorporant de l'EVA et imbibé d'une solution nutritive comprenant, entre autres, un sel d'acide orthophosphorique.

### 1. Préparation du support de culture absorbant et hydrophile

Le support de culture absorbant et hydrophile utilisé est ici une pièce d'Airlaid^{®} provenant de la société SCA, France (référence 95NN81) et ayant un grammage annoncé de 95 g.m⁻², pour une épaisseur de 2 mm.

Dans l'épaisseur de ce matériau en fibres de cellulose ont été immobilisées des particules d'EVA dont la surface a été recouverte d'une composition pulvérulente comprenant un(des) substrat(s) chromogène(s) (par exemple, le 5-bromo-4-chloro-3-indolyl-alpha-glucopyranoside et/ou le 5-bromo-4-chloro-3-indolyl-N-méthyl-*α*-glucopyranoside), de la gomme de xanthane et un agent stimulateur du métabolisme des bactéries (le MnCl₂).

La gomme de xanthane est utilisée pour augmenter quelque peu la capacité d'absorption de l'Airlaid^{®} et pour générer un gel qui facilitera l'implantation et la rétention des bactéries dans l'épaisseur du matériau fibreux. Le MnCl₂ quant à lui permet de stimuler le métabolisme bactérien. Le 5-bromo-4-chloro-3-indolyl-*α*-glucopyranoside et le 5-bromo-4-chloro-3-indolyl-N-méthyl-*α*-glucopyranoside, sous l'action d'hydrolyse d'une *α-*glucosidase, libère des chromophores de couleur bleue/verte visibles à l'oeil nu. Ces substrats chromogènes permettent ainsi de marquer les colonies bactériennes exprimant une activité *α*-glucosidase qui se développeraient sur ce support de culture, comme par exemple des colonies de *S. aureus.*

Plus précisément, les essais ont été réalisés avec des pièces d'Airlaid^{®} carrées, de 2,7 cm de côté. Ces pièces en fibres de cellulose ont été imprégnées à sec d'une composition de poudres comprenant (pour environ 100 g de composition) :
- 80 g d'EVA, avec une granulométrie moyenne de l'ordre de 60-80 µm.
- 20 g de gomme de xanthane, avec une granulométrie moyenne de l'ordre de 20-30 µm.
- 0,533 g 5-bromo-4-chloro-3-indolyl-N-méthyl-*α*-glucopyranoside et 0,3 g de 5-bromo-4-chloro-3-indolyl-*α*-glucopyranoside, avec une granulométrie moyenne de l'ordre de 20-30 µm,
- 0,027 mg de MnCl₂, avec une granulométrie moyenne de l'ordre de 20-30 µm.

Pour faciliter la manipulation de cette composition de poudres, en particulier pour obtenir une bonne répartition homogène de chaque constituant dans le support absorbant, celle-ci a été préalablement soumise à un procédé de mélange à chaud en vue d'agglomérer les particules les unes aux autres.

Grâce à un chauffage et une agitation appropriée, la surface des particules d'EVA est rendue collante et les particules des autres constituants de la composition de poudres viennent y adhérer. Le chauffage est réalisé à une température de l'ordre de 50-60°C, qui entraine le ramollissement des particules d'EVA et rend leur surface collante. Le mélange est réalisé de façon mécanique.

Cette composition de poudres ainsi assemblée à chaud renferme des particules d'EVA, plus ou moins bien individualisées, dont la surface se trouve en partie tapissée de particules de 5-bromo-4-chloro-3-indolyl-N-méthyl-*α*-glucopyranoside et 5-bromo-4-chloro-3-indolyl-α-glucopyranoside, de particules de gomme de xanthane et de particules de MnCl₂. Elle est ensuite transférée dans l'épaisseur d'une feuille d'Airlaid^{®} par une technique d'imprégnation à sec telle que par exemple décrite dans WO 2015/044605, WO 2010/001043 et WO 99/22920. Cette technique consiste à saupoudrer la feuille d'Airlaid^{®} de la composition de poudres et à mettre les particules de ladite composition en vibration sous l'action d'un champ électrique alternatif; les particules pénètrent et s'enfoncent alors progressivement dans les cavités du corps fibreux.

Une fois imprégnée, la feuille d'Airlaid^{®} est calandrée entre deux feuilles de papier sulfurisé, la face imprégnée étant tournée du côté du plateau chauffant. Ce calandrage est réalisé sous 4 bars de pression et à 80°C, pendant 1-2 minutes.

La feuille d'Airlaid^{®} est découpée en pièces de 2,7 cm de côtés. Chacun de ces carrés d'Airlaid^{®} renferme environ 0,06 g de poudre, renfermant environ 80 % d'EVA.

### 2. Souches bactériennes utilisées et préparation de la solution nutritive

Des souches de *Staphylococcus aureus* ont été utilisées, en l'occurrence une souche sensible à la méthicilline (MSSA) provenant de la collection de la Demanderesse. Dans le cadre de ce test, les nutriments nécessaires à la croissance et au développement de ces bactéries sont apportées par du sérum humain (provenant de l'EFS) dilué au PBS. Cette solution nutritive vise à reproduire les qualités nutritionnelles des exsudats de plaies.

Diverses compositions d'exsudats synthétiques ou reconstituées sont décrites dans la littérature scientifique et peuvent être utilisées pour reproduire le test mené par les inventeurs. Un bouillon de culture de qualités nutritionnelles faibles ou encore une eau peptonée peuvent aussi être utilisés à cette même fin.

Pour évaluer l'effet de la concentration de KH₂PO₄ sur la croissance et le développement des bactéries soumises à la présence de l'EVA (contenu ici dans l'épaisseur de la pièce d'Airlaid^{®}), le sérum dilué au PBS est additionné de KH₂PO₄ : 0 g.L⁻¹, 3 g.L⁻¹, 4 g.L⁻¹, 5 g.L⁻¹ et 6 g.L⁻¹ (en concentrations finales).

### 3. Mise en oeuvre du test et résultats obtenus

100 µL d'une préparation cellulaire concentrée à 10⁶ UFC.mL⁻¹ (dans du trypton sel) sont ajoutés à 900 µL de sérum dilué au PBS, avec ou sans KH₂PO₄. Les pièces d'Airlaid^{®} sont trempées dans cette solution puis incubées en jarre pendant 17 heures à 32°C, avec un peu d'eau dans la jarre pour éviter le dessèchement.

Après incubation, les pièces de support de culture sont examinées et les constatations suivantes ont pu être faites :
- Les pièces d'Airlaid^{®} ensemencées avec des bactéries et un sérum dilué au PBS, dépourvu de KH₂PO₄ sont incolores. Aucune colonie n'y a poussé.
- Une teinte de couleur verte est observée sur les pièces ensemencées avec des bactéries et un sérum dilué au PBS complémenté avec du KH₂PO₄. Une coloration particulièrement marquée est constatée pour les concentrations de KH₂PO₄ de 3 g.L⁻¹ et 5 g.L⁻¹. Cette coloration est encore plus intense à 4 g.L⁻¹ de KH₂PO₄. A 6 g.L⁻¹ de KH₂PO₄, elle est à peine observable.

### C. Démonstration en milieu de culture liquide

L'évaluation des effets de l'EVA et des sels d'acide orthophosphorique sur la croissance et le développement bactériens a également été réalisée en milieu liquide. L'objectif est d'évaluer plus particulièrement l'impact de l'EVA et du KH₂PO₄ sur la cinétique de croissance de bactéries.

A cet effet, 1 mL d'une suspension de *Staphylococcus aureus* (300 UFC.mL⁻¹) provenant de la collection de la Demanderesse a été cultivé à 37°C dans 9 mL de différentes solutions nutritives :
- du sérum dilué au PBS,
- du sérum dilué au PBS, et comprenant 18,5 g.L⁻¹ d'EVA,
- du sérum dilué au PBS, et comprenant 18,5 g.L⁻¹ d'EVA et 4 g.L⁻¹ de KH₂PO₄.

Après chaque heure écoulée, un comptage de cellules est effectué pour un tube de culture de chaque série. Pour ce faire, les cellules de chaque tube sont repiquées sur une boîte d'agar chromID^{®} S. aureus (bioMérieux, France). Les boîtes sont incubés 24 heures à 37°C, avant de compter les colonies formées.

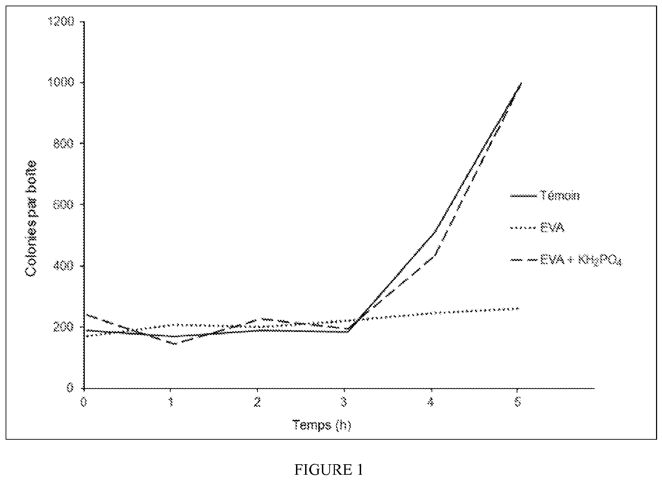

Les trois cinétiques de croissance ainsi mesurées sont présentées sous forme de courbes à la Figure 1.

Ces résultats confirment ceux obtenus précédemment, sur un milieu de culture solide. L'EVA a un effet bactériostatique. Cet effet bactériostatique peut être inhibé par des sels d'acide orthophosphorique, tels que KH₂PO₄. Utiliser le KH₂PO₄ en quantité pondérale correspondant à de l'ordre de 1/5 de la quantité d'EVA, permet de contrebalancer efficacement l'effet bactériostatique de l'EVA.

### Exemple 2 : Préparation de biocapteurs selon l'invention et évaluation de leur capacité à la détection et à l'identification de bactéries

### A. Préparation et assemblage

L'exemple suivant illustre la réalisation d'un biocapteur selon l'invention, suivant un mode particulier.

Dans ce mode particulier, ledit biocapteur est préparé à partir d'une pièce réalisée en matériau fibreux absorbant hydrophile. Dans l'épaisseur de ce matériau et fixées à ses fibres, des particules EVA retiennent et exposent à leur surface un mélange pulvérulent associant :
- un sel d'acide orthophosphorique (du KH₂PO₄),
- deux substrats chromogènes d'α-glucosidase (le 5-bromo-4-chloro-3-indolyl-N-méthyl-*α*-glucopyranoside et le 5-bromo-4-chloro-3-indolyl-*α*-glucopyranoside)
- un gélifiant (de la gomme de xanthane), et
- un activateur de métabolisme bactérien (du MnCl₂).

Les particules d'EVA revêtues dudit mélange pulvérulent sont préalablement préparées en mélangeant à chaud une composition pulvérulente de particules dissociées, comprenant (pour environ 100 g de composition) :
- 80 g d'EVA,
- 20 g de gomme de xanthane
- 6,67 g de KH₂PO₄,
- 0,533 g de 5-bromo-4-chloro-3-indolyl-N-méthyl-*α*-glucopyranoside et 0,3 g de 5-bromo-4-chloro-3-indolyl-*α*-D-glucopyranoside, et
- 0,027 g de MnCl₂.

Ce mélange pulvérulent est soumis à un procédé de mélange à chaud en vue d'obtenir une composition de poudres agglomérées, mis en oeuvre dans les mêmes conditions que celles du mélange à chaud précédemment décrit.

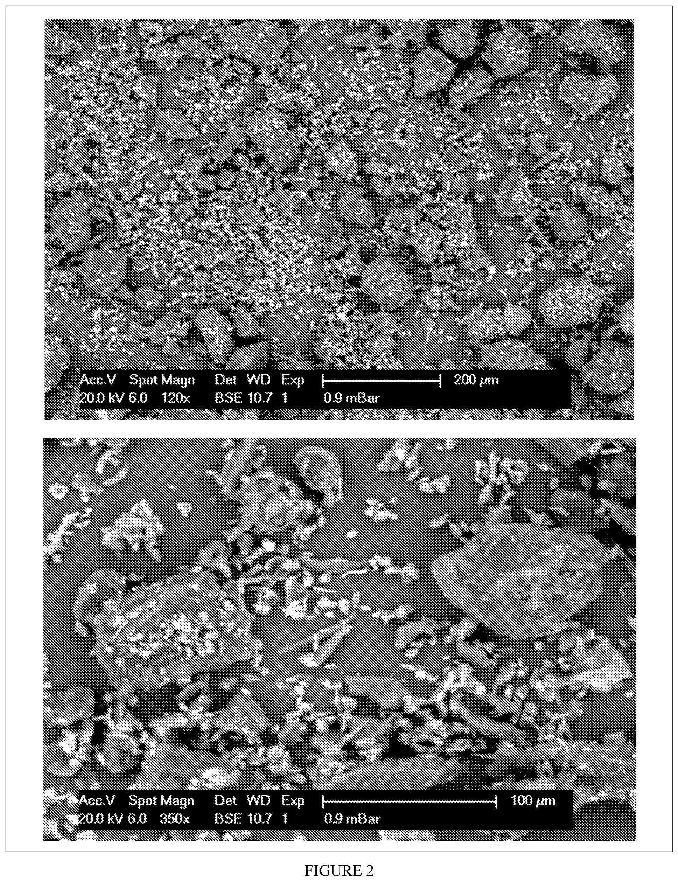

La figure 2 montre deux photographies prises en microscopie électronique à balayage de la composition pulvérulentes de particules dissociées, avant le mélange à chaud.

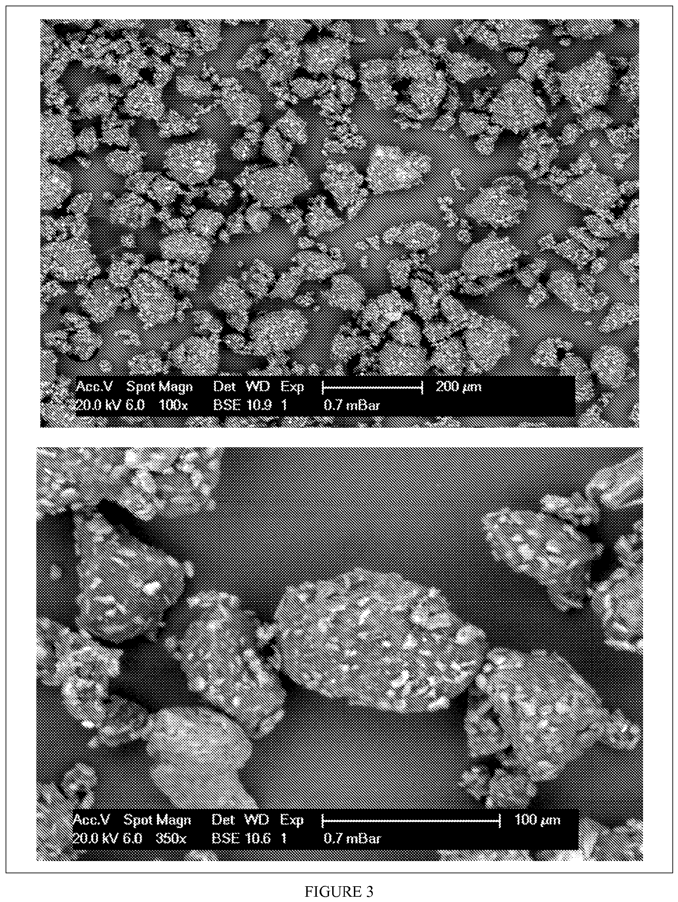

La figure 3 montre deux photographies prises en microscopie électronique à balayage d'une composition de poudres agglomérées selon l'invention, dans laquelle on distingue clairement des particules d'EVA, à la surface desquelles se répartissent de plus fines particules, en l'occurrence des particules de KH₂PO₄, de 5-bromo-4-chloro-3-indolyl-N-méthyl-*α*-glucopyranoside et 5-bromo-4-chloro-3-indolyl-N-méthyl-*α*-glucopyranoside, de gomme de xanthane et de MnCl₂.

La composition de poudres agglomérées ainsi préparée est ensuite transférée dans l'épaisseur d'une feuille d'Airlaid ^{®} par une technique d'imprégnation à sec, comme précédemment détaillé.

Une fois imprégnée, la feuille d'Airlaid^{®} est calandrée entre deux feuilles de papier sulfurisé, la face imprégnée étant tournée du côté du plateau chauffant. Ce calandrage est réalisé sous 4 bars de pression et à 80°C, pendant environ 1 minute et 30 secondes.

Des biocapteurs selon l'invention, sont découpés à partir de cette feuille d'Airlaid^{®} imprégnée à sec de la composition de poudres agglomérées, puis calandrée. Ils permettent la détection de bactéries exprimant une activité *α*-glucosidase, comme cela est le cas notamment pour les bactéries *Staphylococcus aureus.*

D'autres biocapteurs de conception très proche au premier exemple ont également été assemblés. Dans ces biocapteurs selon l'invention, les particules d'EVA exposent à leur surface également de la céfoxitine, un antibiotique à l'égard duquel les souches de *Staphylococcus aureus* sensibles à la méthicilline (MSSA) sont particulièrement sensibles, à la différence des souches résistantes à la méthicilline (MRSA). Ce deuxième exemple de biocapteurs selon l'invention permet la détection et l'identification de souches de MRSA.

La céfoxitine est fixée sur la surface des particules d'EVA de façon concomitante avec le KH₂PO₄, le 5-bromo-4-chloro-3-indolyl-N-méthyl-*α*-glucopyranoside et/ou le 5-bromo-4-chloro-3-indolyl-*α*-glucopyranoside, la gomme de xanthane et le MnCl₂, par un procédé de mélange à chaud. Pour ce faire, la composition pulvérulente de particules dissociées précédemment énoncée comprend également 6,6 mg de céfoxitine.

### B. Evaluation des biocapteurs précédents pour la détection et l'identification de Staphylococcus aureus

Des biocapteurs tels que précédemment décrits et embarquant une composition de poudres agglomérées selon l'invention, comprenant des particules d'EVA sur la surface de laquelle se répartissent :
- KH₂PO₄,
- 5-bromo-4-chloro-3-indolyl-N-méthyl- *α*-glucopyranoside
- 5-bromo-4-chloro-3-indolyl-*α*-glucopyranoside,
- gomme de xanthane et
- MnCl₂,
ont été testés pour leur aptitude à la détection de souches de *Staphylococcus aureus,* en l'occurrence des souches sensibles à la méthicilline (MSSA) provenant de la collection de la Demanderesse.

Pour ce faire, sur des carrés de biocapteurs, de 2,7 cm de côté, a été déposé 1 mL de sérum dilué au PBS, comprenant 10⁵ UFC d'une souche de MSSA (ou 1 mL de sérum dilué au PBS exempte de bactéries, pour les biocapteurs contrôles).

Les pièces d'Airlaid^{®} sont alors incubées en jarre pendant 17 heures à 32°C, avec un peu d'eau dans la jarre pour éviter le dessèchement.

Après incubation, les biocapteurs sont examinés et les constatations suivantes ont pu être faites :
- Les biocapteurs contrôles ensemencés uniquement avec du sérum dilué au PBS restent incolores.
- Une teinte de couleur verte est observée sur les biocapteurs sans céfoxitine, ensemencés avec des bactéries MSSA et le sérum dilué au PBS.
- Les biocapteurs renfermant de la céfoxitine et ensemencés avec des bactéries MSSA et le sérum dilué au PBS, restent incolores.

## Revendications

1. Biocapteur comprenant une pièce en matériau absorbant et hydrophile fixant, en surface et/ou dans son épaisseur, une composition de poudres agglomérées comprenant des particules d'éthylène-acétate de vinyle (EVA) ayant une surface en partie recouverte au moins d'un sel d'acide orthophosphorique et d'un indicateur visuel de croissance microbiologique.

2. Biocapteur selon la revendication 1, dans lequel le sel d'acide orthophosphorique est choisi parmi le dihydrogénophosphate de potassium (KH₂PO₄) et le dihydrogénophosphate de sodium (NaH₂PO₄).

3. Biocapteur selon la revendication 1 ou 2, dans lequel l'EVA et le KH₂PO₄ sont présents dans un rapport pondéral KH₂PO₄/EVA compris entre 1:25 et 1:8.

4. Biocapteur selon la revendication 1 ou 2, dans lequel l'EVA et le NaH₂PO₄ sont présents dans un rapport pondéral NaH₂PO₄/EVA compris entre 1:15 et 1:3.

5. Biocapteur selon l'une quelconque des revendications 1 à 4, dans lequel l'EVA présente une teneur pondérale en acétate de vinyle de 10 à 40 %.

6. Biocapteur selon l'une quelconque des revendications 1 à 5, dans lequel la surface des particules d'EVA est également recouverte d'un agent gélifiant.

7. Biocapteur selon la revendication 6, dans lequel l'agent gélifiant est choisi parmi l'agar, l'agarose, la gomme de guar et la gomme de xanthane.

8. Biocapteur selon l'une quelconque des revendications 1 à 7, dans lequel la surface des particules d'EVA est également recouverte de MnCl₂.

9. Biocapteur selon l'une quelconque des revendications 1 à 8, dans lequel la surface des particules d'EVA est également recouverte d'un agent sélectif aux effets antibiotiques et/ou antifongiques.

10. Biocapteur selon l'une quelconque des revendications 1 à 9, dans lequel la pièce en matériau absorbant et hydrophile est une pièce de non-tissé.

11. Composition de poudres agglomérées comprenant des particules d'EVA ayant une surface en partie recouverte au moins d'un sel d'acide orthophosphorique et d'un indicateur visuel de croissance microbiologique, dans laquelle le sel d'acide orthophosphorique est le dihydrogénophosphate de potassium (KH₂PO₄) et dans laquelle l'EVA et le KH₂PO₄ sont présents dans un rapport pondéral KH₂PO₄/EVA compris entre 1:25 et 1:8.

12. Composition de poudres agglomérées comprenant des particules d'EVA ayant une surface en partie recouverte au moins d'un sel d'acide orthophosphorique et d'un indicateur visuel de croissance microbiologique, dans laquelle le sel d'acide orthophosphorique est le dihydrogénophosphate de sodium (NaH₂PO₄) et dans laquelle l'EVA et le NaH₂PO₄ sont présents dans un rapport pondéral NaH₂PO₄/EVA compris entre 1:15 et 1:3.

## Patentansprüche

1. Biosensor, der ein Stück aus einem absorbierenden und hydrophilen Material umfasst, das auf der Oberfläche und/oder in seiner Dicke eine Zusammensetzung von agglomerierten Pulvern bindet, die Ethylen-Vinylacetat-(EVA-) Partikel mit einer Oberfläche umfassen, die teilweise mit mindestens einem Orthophosphorsäuresalz und einem visuellen Indikator für ein mikrobiologisches Wachstum beschichtet ist.

2. Biosensor nach Anspruch 1, wobei das Orthophosphorsäuresalz aus Kaliumdihydrogenphosphat (KH₂PO₄) und Natriumdihydrogenphosphat (NaH₂PO₄) ausgewählt ist.

3. Biosensor nach Anspruch 1 oder 2, wobei das EVA und das KH₂PO₄ in einem KH₂PO₄:EVA-Gewichtsverhältnis zwischen 1:25 und 1:8 vorhanden sind.

4. Biosensor nach Anspruch 1 oder 2, wobei das EVA und das NaH₂PO₄ in einem NaH₂PO₄ :EVA-Gewichtsverhältnis zwischen 1:15 und 1:3 vorhanden sind.

5. Biosensor nach einem der Ansprüche 1 bis 4, wobei das EVA einen Vinylacetat-Gehalt von 10 bis 40 Gew.-% aufweist.

6. Biosensor nach einem der Ansprüche 1 bis 5, wobei die Oberfläche der EVA-Partikel auch mit einem Geliermittel beschichtet ist.

7. Biosensor nach Anspruch 6, wobei das Geliermittel aus Agar, Agarose, Guar-Gummi und Xanthan-Gummi ausgewählt ist.

8. Biosensor nach einem der Ansprüche 1 bis 7, wobei die Oberfläche der EVA-Partikel auch mit MnCl₂ beschichtet ist.

9. Biosensor nach einem der Ansprüche 1 bis 8, wobei die Oberfläche der EVA-Partikel auch mit einem Selektionsmittel für antibiotische und/oder fungizide Wirkungen beschichtet ist.

10. Biosensor nach einem der Ansprüche 1 bis 9, wobei es sich bei dem Stück aus einem absorbierenden und hydrophilen Material um ein Vliesstoffstück handelt.

11. Zusammensetzung von agglomerierten Pulvern, die EVA-Partikel umfassen, deren Oberfläche teilweise mit mindestens einem Orthophosphorsäuresalz und einem visuellen Indikator für ein mikrobiologisches Wachstum beschichtet ist, wobei das Orthophosphorsäuresalz Kaliumdihydrogenphosphat (KH₂PO₄) ist und wobei das EVA und das KH₂PO₄ in einem KH₂PO₄:EVA-Gewichtsverhältnis zwischen 1:25 und 1:8 vorhanden sind.

12. Zusammensetzung von agglomerierten Pulvern, die EVA-Partikel umfassen, deren Oberfläche teilweise mit mindestens einem Orthophosphorsäuresalz und einem visuellen Indikator für ein mikrobiologisches Wachstum beschichtet ist, wobei das Orthophosphorsäuresalz Natrumdihydrogenphosphat (NaH₂PO₄) ist und wobei das EVA und das NaH₂PO₄ in einem NaH₂PO₄ :EVA-Gewichtsverhältnis zwischen 1:15 und 1:3 vorhanden sind.

## Claims

1. A biosensor comprising a piece of absorbent hydrophilic material on which, on the surface and/or within the thickness thereof, a composition of agglomerated powders is immobilized, said composition comprising ethylene-vinyl acetate (EVA) particles having a surface partially coated at least with a salt of orthophosphoric acid and a visual indicator of microbiological growth.

2. The biosensor as claimed in claim 1, in which the salt of orthophosphoric acid is selected from potassium dihydrogen phosphate (KH₂PO₄) and sodium dihydrogen phosphate (NaH₂PO₄).

3. The biosensor as claimed in claim 1 or 2, in which EVA and KH₂PO₄ are present in a KH₂PO₄/EVA weight ratio between 1:25 and 1:8.

4. The biosensor as claimed in claim 1 or 2, in which EVA and NaH₂PO₄ are present in an NaH₂PO₄/EVA weight ratio between 1:15 and 1:3.

5. The biosensor as claimed in any one of claims 1 to 4, in which the EVA has a vinyl acetate content by weight from 10 to 40%.

6. The biosensor as claimed in any one of claims 1 to 5, in which the surface of the EVA particles is also coated with a gelling agent.

7. The biosensor as claimed in claim 6, in which the gelling agent is selected from agar, agarose, guar gum and xanthan gum.

8. The biosensor as claimed in any one of claims 1 to 7, in which the surface of the EVA particles is also coated with MnCh.

9. The biosensor as claimed in any one of claims 1 to 8, in which the surface of the EVA particles is also coated with a selective agent for antibiotic and/or antifungal effects.

10. The biosensor as claimed in any one of claims 1 to 9, in which the piece of absorbent hydrophilic material is a piece of nonwoven.

11. A composition of agglomerated powders comprising EVA particles having a surface partially coated at least with a salt of orthophosphoric acid and a visual indicator of microbiological growth, in which the salt of orthophosphoric acid is potassium dihydrogen phosphate (KH₂PO₄), and in which EVA and KH₂PO₄ are present in a KH₂PO₄/EVA weight ratio between 1:25 and 1:8.

12. A composition of agglomerated powders comprising EVA particles having a surface partially coated at least with a salt of orthophosphoric acid and a visual indicator of microbiological growth, in which the salt of orthophosphoric acid is potassium dihydrogen phosphate (KH₂PO₄), and in which EVA and KH₂PO₄ are present in a KH₂PO₄/EVA weight ratio between 1:15 and 1:3.
